# EUROPEAN PATENT SPECIFICATION

(11) **EP 2 579 885 B1**
(45) Date of publication and mention of the grant of the patent: **12.10.2016**
(21) Application number: 11730197.8
(22) Date of filing: 09.06.2011
(51) Int. Cl.: A61K 35/76, A61P 35/00, A61K 48/00

(54) **PARVOVIRUS FOR TREATMENT OF TUMOURS BY INTRANASAL APPLICATION**
PAROVIRUS ZUR BEHANDLUNG VON TUMOREN MITTELS INTRANASALER ANWENDUNG
PARVOVIRUS POUR LE TRAITEMENT DE TUMEURS PAR APPLICATION INTRANASALE

(30) Priority: 11.06.2010 EP 10006082
(43) Date of publication of application: 17.04.2013
(73) Proprietor: Deutsches Krebsforschungszentrum, 69120 Heidelberg (DE)
(72) Inventor: ROMMELAERE, Jean, 69118 Heidelberg (DE); KIPRIJANOVA, Irina, 69118 Heidelberg (DE); SCHLEHOFER, Jörg, 69181 Leimen (DE); AYACHE, Ali, 69120 Heidelberg (DE); FISCHER, Manuel, 69151 Neckargemünd (DE)
(74) Representative: Schüssler, Andrea
(86) International application number: PCT/EP2011/002842
(87) International publication number: WO 2011/154146

(56) References cited:
- WO-A2-2006/047301
- US-A1- 2004 220 124
- ROMMELAERE J ET AL: "Oncolytic parvoviruses as cancer therapeutics", CYTOKINE AND GROWTH FACTOR REVIEWS, ELSEVIER LTD, GB, vol. 21, no. 2-3, 1 April 2010 (2010-04-01), pages 185-195, XP027066640, ISSN: 1359-6101, DOI: DOI:10.1016/J.CYTOGFR.2010.02.011 [retrieved on 2010-03-07]
- "186 POSTER Complete remission of advanced autologous intracranial gliomas by oncolytic Parvovirus H-1", EUROPEAN JOURNAL OF CANCER. SUPPLEMENT, PERGAMON, OXFORD, GB, vol. 3, no. 2, 1 October 2005 (2005-10-01) , page 53, XP005132303, ISSN: 1359-6349
- RAYKOV ZAHARI ET AL: "Carrier cell-mediated delivery of oncolytic parvoviruses for targeting metastases", INTERNATIONAL JOURNAL OF CANCER, JOHN WILEY & SONS, INC, UNITED STATES, SWITZERLAND, GERMANY, vol. 109, no. 5, 1 May 2004 (2004-05-01), pages 742-749, XP002554386, ISSN: 0020-7136, DOI: DOI:10.1002/IJC.20013
- IN-KWON HAN ET AL: "Enhanced brain targeting efficiency of intranasally administered plasmid DNA: an alternative route for brain gene therapy", JOURNAL OF MOLECULAR MEDICINE, SPRINGER, BERLIN, DE, vol. 85, no. 1, 7 November 2006 (2006-11-07), pages 75-83, XP019467083, ISSN: 1432-1440, DOI: DOI:10.1007/S00109-006-0114-9

## Description

The present invention relates to a parvovirus for use in a method of treatment of a tumor and/or tumor metastases by intranasal administration, wherein the tumor is a brain tumor and/or the metastases are cerebral metastases.

Rodent parvoviruses are single-stranded DNA viruses possessing intrinsic oncolytic activity, i.e. they preferentially replicate in and kill cancer cells of both murine and human origin. In this regard, oncolytic parvoviruses, in particular rodent parvoviruses represent novel tools for cancer treatment. Though the virus can enter most nontransformed (normal) cells, infection is abortive and fails to produce progeny virions. In contrast, in many malignant cells or in cells transformed with various means, parvovirus infection leads to cell killing which is not observed in the corresponding nontransformed cells, and can be productive. The enhanced permissiveness of transformed cells for parvoviruses was shown to be due to at least in part to changes in factors associated with (i) cell cycling and differentiation and (ii) controlling viral DNA replication and gene expression. Besides specifically killing cancer cells (oncolysis), these parvoviruses also provide danger signals prompting the immune system to eliminate virus-infected tumours. As a consequence of oncolytic events, the innate and adaptive immune systems gain access to tumour antigens, which results in cross-priming and vaccination effects.

Glioblastoma is a devastating disease with only limited treatment possibilities. Evidently, the prognosis for patients requires new therapies. The replication competent oncolytic viruses discussed above are considered promising since they are able to spread through malignant tissues and induce antitumor immune-responses. Among them, rodent parvoviruses appear to be excellent candidates, due to their natural oncotropism, their capacity to infect human cells, the specific toxicity for neoplastic cells, and the low pathogenicity for humans. In a rat model, H1-PV was able to cause a complete regression of established gliomas without any relaps and the viruses proved able to target various human gliomas, thereby efficiently killing these cells irrespective of their acquired resistance towards known death inducers. However, the routes of administration of parvovirus as regards efficiency and convenience are in need of improvement.

US 2004/0220124 A1 discloses a method of treating a brain tumor in a patient by intravenous, intraperitoneal, subcutaneous, intramuscular, topical, intradermal or intratumoral administration of a parvovirus.

Therefore, it is the object of the present invention to provide a route of administration for oncolytic parvoviruses overcoming the drawbacks of the prior art.

According to the invention this is achieved by the subject matters defined in the claims.

It was surprisingly found that intranasally administered parvoviruses can successfully be used for highly efficient suppression of cerebral gliomas in rats. In the study leading to the present invention, the concept of H-1PV-based virotherapy of glioma by intranasal application of H-1PV was tested for rat (RG-2 cell-derived) gliomas in immunocompetent rat models. Rats were treated with intranasally administered H-1PV. Tumor regression was monitored by magnetic resonance imaging, and oncolysis was proven by histology. Presence of viral DNA and viral capsids were detected in the brain. Virus replication in tumors may contribute to secondary infection by progeny virus to the efficiency of oncolysis. In immunocompetent rats bearing RG-2 derived tumors, a single intranasal application of H-1PV of the virus was sufficient for remission of advanced and even symptomatic intracranial gliomas without damaging normal brain tissue or other organs. Moreover, H-1PV therapy resulted in significantly improved survival (Kaplan-Meier analysis) in the rat glioma models. The results presented in the examples, below, and the innocuousness of H-1PV for humans argue for the use of H-1PV as a powerful means to perform oncolytic therapy of tumors, in particular malignant gliomas by intranasal application of the parvovirus.

To summarize, intranasal parvotherapy according to the present invention is useful for the therapy of tumors, in particular brain tumors, and can improve the prognosis of said tumors. Parvovirus H1 infection leads to killing of tumor cells but does not harm normal brain cells. Thus, the intranasal use of a parvovirus, like H1, or vectors based on such a virus offers the chance of tumor specific therapy without severe neurological side effects. Advantages of intranasal application would facilitate also multiple applications (if required), e.g. by using a spray. In contrast to parenteral applications, intranasal application would not readily induce serum antibodies, and if serum antibodies might be present, intranasaly applied virus would not be readily neutralized. It is conceivable that also brain metastases of tumors of other than glial origin may be reached by the oncolytic parvovirus.

### Brief description of the drawings

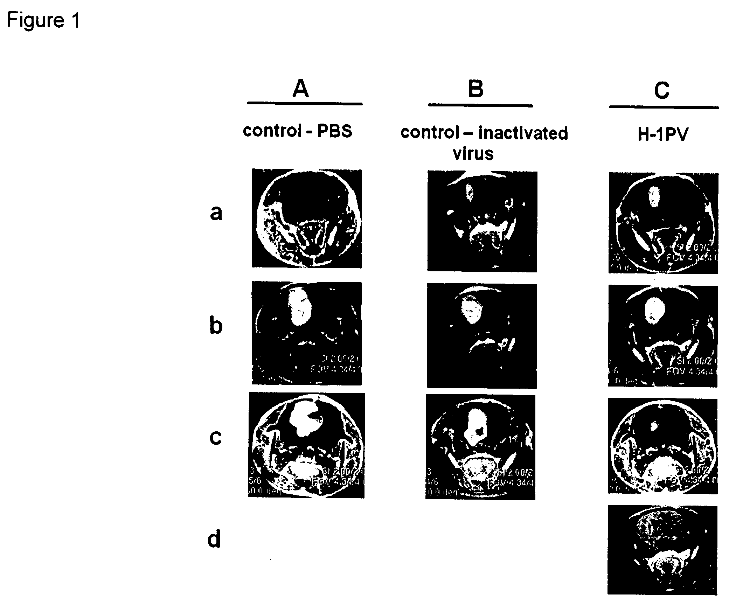

Figure 1: Glioma regression by intranasally administered H-1PV MR images of three animals, each from one experimental group, are given as representative examples. Rats bearing an intracranial RG-2 glioma were intranasally treated with **(A)** PBS, **(B)** noninfectious H-1PV capsids (UV-irradiated empty capsids equivalent to 1x10¹⁰ particles), **(C)** infectious H-1PV (1 x 10⁸ pfu) 7 days after tumor cell implantation. MR imaging was performed 7 (a), 12 (b), 17 (c) or 22 (d) days after tumor cell implantation. There are no images at day 22 (d) for the control groups since no PBS- or empty virus-treated rats survived for so long. MR image for group B (empty capsids) had to be performed at day 16 due to the lack of survivors at later times. The group C animals survived symptom-free for more than 80 days after tumor cell implantation. NB: Probably due to regression of the tumor, MRI shape of brains changed slightly (C, d).

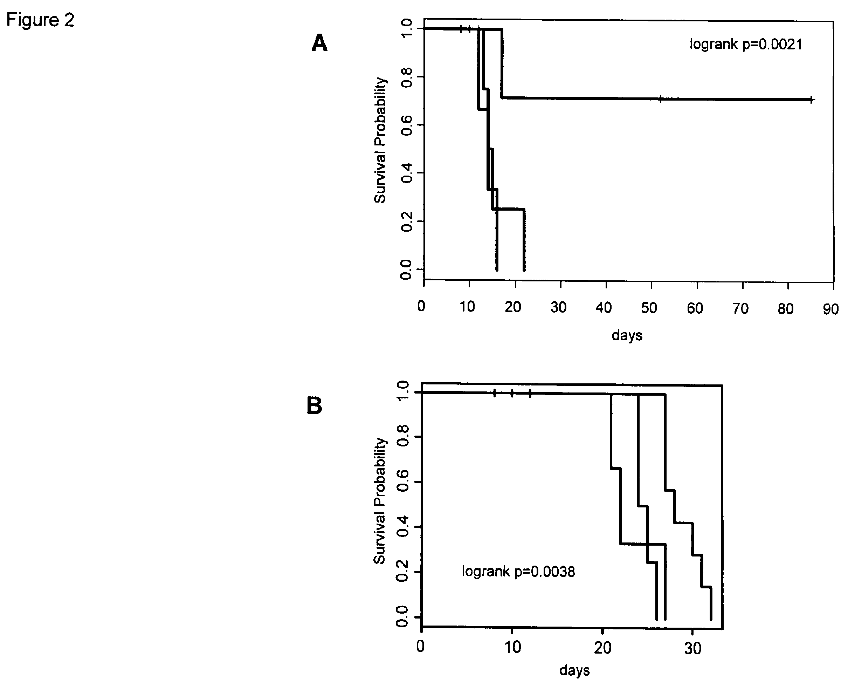

Figure 2: Kaplan-Meier analysis of the survival rate of glioma-bearing rats after intranasal instillation of H-1PV
   **(A)** RG-2 glioma-bearing Wistar rats;
   **(B)** U87 glioma-bearing RNU rats. The x-axis shows days after tumor cell implantation. Vertical bars indicate censoring of animals for analysis purposes. Red line: PBS (A: n=6; B: n=5), black line: empty H-1PV capsids (A: n=5; B: n=7), green line: infectious H-1PV virions (A: n=15; B: n=11). The difference between the H-1PV-treated group and the controls was statistically significant in both models (log rank test: A: p=0.0021; B: p=0.0038). (In the Wistar rat group, 17 days after tumor cell implantation, 2 animals of the H-1PV-infected group died from tumor unrelated reasons.)

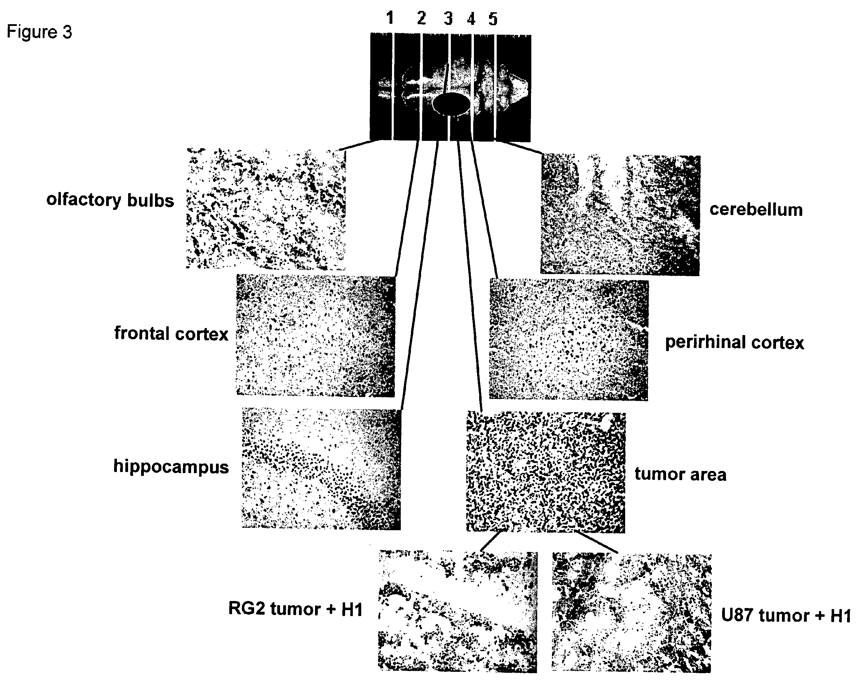

Figure 3: Histology of brains of H-1PV-infected animals Haematoxylin-Eosin staining of sections from brain areas as indicated, 5 days after intranasal infection with H-1PV. Morphology of infected brains was not altered after infection (conformed by a neuropathologist) except for the glioma area in tumor bearing animals (RG-2 cell-derived tumor in Wistar rats or U87 cell-derived tumor in RNU rats, as indicated). Five days after infection, destruction of tumor cells was more pronounced in RG-2 glioma compared with U87 glioma.

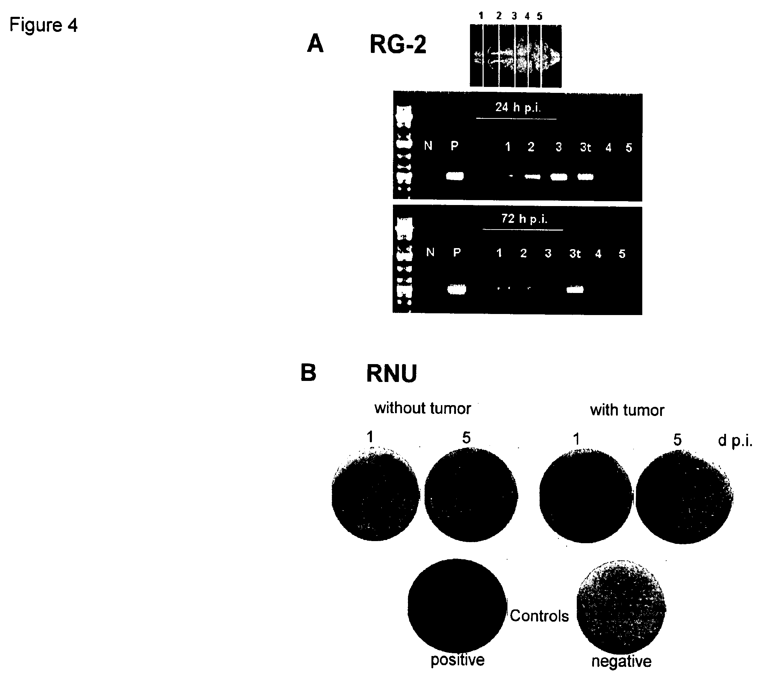

Figure 4: Presence_of viral DNA in brains of glioma-bearing animals intranasally infected with H-1PV
   **(A)** Detection of viral DNA in different sections of brains of RG-2 cell glioma bearing rats. Section positions within the brain are indicated: 1: olfactory bulbs, 2: frontal cortex/caudate putamen area, 3: tumor area/hippocampus, 4: perirhinal cortex/subiculum, 5: cerebellum. DNA was extracted from these sections at 24 h or 72 h after infection and viral DNA was detected by PCR. The amplified product (516 bp) was visualized after agarose gel electrophoresis. The glioma was localized in section 3, which was divided in tumor-containing (3t) and -free (3) parts. N=negative control (tissue from mock-infected animals); P=positive control (DNA from H-1PV-infected RG-2 cells).
   **(B)** Dispersed cell assay of brains from infected immunodeficient RNU rats. NBK cells were inoculated with sterile filtered brain tissue of infected animals and transferred to a membrane followed by hybridization with a NS-1-specific radioactive labeled probe. Results from the tumor bearing hemisphere of the brain and the corresponding hemisphere of a non-tumor bearing animal are shown for comparison, 1 and 5 days p.i.. Controls included H-1PV infected cells and mock-infected cells.

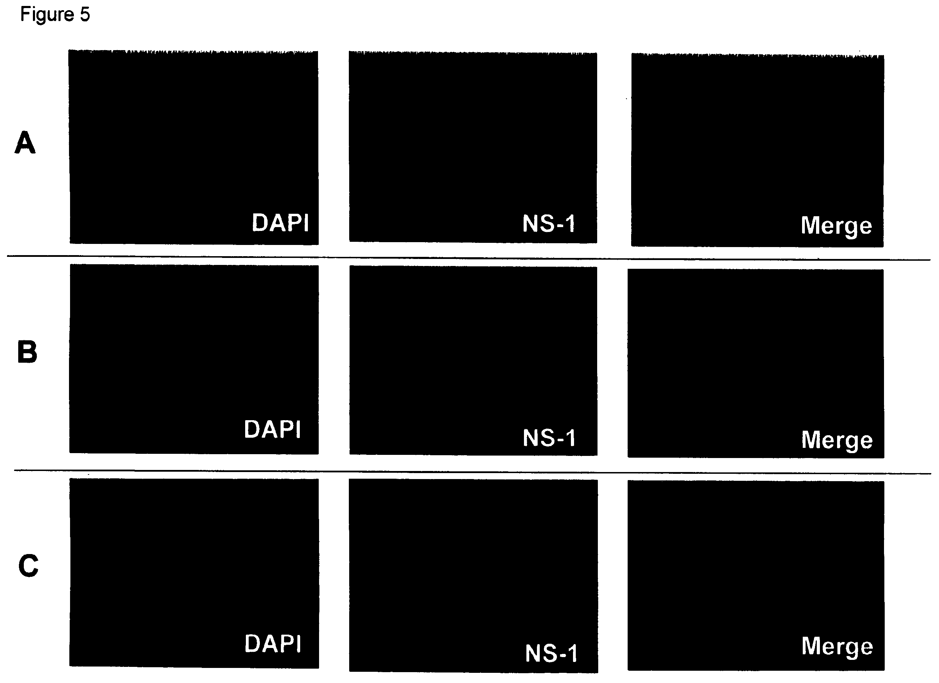

Figure 5: Restriction of expression H-1PV non-structural protein 1 (NS-1) to the glioma area of rat brains after intranasal infection
   Five days p.i., expression of NS-1 was detected in brain sections by immunohistochemistry, using anti-NS-1 antibodies and DAPI counterstaining as indicated.
   **(A)** tumor area of a RG-2 glioma-bearing brain (NS-1 signal, red fluorescence);
   **(B)** tumor area of a U87 glioma-bearing brain (NS-1 signal, green fluorescence);
   **(C)** non-tumor area of the brain of an infected RG-2 glioma-bearing rat (also in U87 glioma-bearing infected RNU rats, there was no NS-1 signal in the non-tumor areas of the brain).

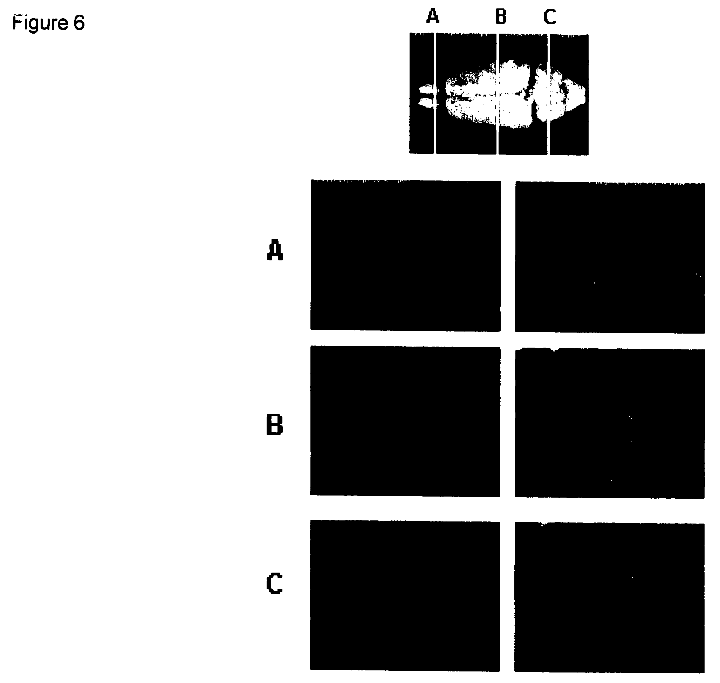

Figure 6: Detection of H-1PV capsid proteins in rat brain after intranasal instillation of H-1PV
   A representative Wistar rat was sacrificed 10 days after RG-2 cell implantation (i.e. 3 days after intranasal virus instillation). H-1PV capsid proteins (VP) were detected by immunostaining of brain sections using VP-specific antiserum and DAPI counterstaining (VP, green signals).
   **(A)** area of olfactory bulbs,
   **(B)** tumor area,
   **(C)** cerebellum. Similar results were obtained in sections from brains bearing a U87 cell-derived tumor and in non-tumor-bearing rats. VP could also be detected after instillation of empty viral capsids.

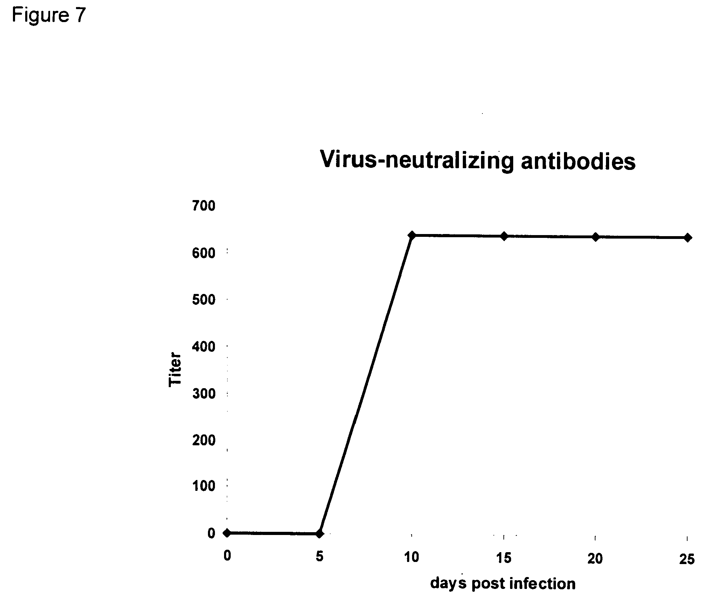

Figure 7: Appearance of H-1PV-neutralizing serum antibodies after intranasal virus infection
   Blood samples were obtained from a representative animal (Wistar rat) at different days after H-1PV infection (as indicated), and virus-neutralizing antibody titers were determined by MTT test. Titers are given as reciprocals of serum dilution, defined as the dilution, which reduced the viral cytotoxic effect of H-1PV by 50%.

The present invention relates to a parvovirus for use in a method of treatment of a tumor and/or tumor metastases by intranasal administration, wherein the tumor is a brain tumor and/or the metastases are cerebral metastases.

The term "parvovirus" as used herein comprises wild-type or modified replication competent derivatives thereof as well as vectors, e.g. expression vectors, based on such viruses or derivatives. A person skilled in the art is familiar with examples thereof. In a preferred embodiment, the vector containing the DNA of a parvovirus is a virus, e.g. an adenovirus, vaccinia virus, an AAV virus or a parvovirus, such as MVM or H-1, a parvovirus being preferred. The vector may also be a retrovirus, such as MoMULV, MoMuLV, HaMuSV, MuMTV, RSV or GaLV. Moreover, the parvovirus can be applied as naked DNA or can be coupled to, or embedded within, a liposome surface, thus allowing enhanced delivery.

For constructing (expression) vectors which contain parvoviral DNA, it is possible to use general methods known in the art. These methods include, e.g., in vitro recombination techniques, synthetic methods and in vivo recombination methods.

Suitable parvoviruses, derivatives etc. are known to the person skilled in the art.

Preferably, parvoviruses are used that are capable of propagating and spreading through human tumor cells. The term "propagating" as used herein means that the progeny virions of the parvovirus variant show amplification significantly over input levels (i.e. > 5 fold). The term "spreading through" as used herein means that the progeny virions of the parvovirus variant are capable not only of killing primarily infected cell populations, but are able to cause secondary infections of naive cells with particles produced during the first round of infection.

Preferably, the parvovirus used in the present invention is a rodent parvovirus with rat parvovirus being particularly preferred or a related rodent parvovirus. Examples of preferred related rodent parvovirus are LuIII, Mouse minute virus (MMV), Mouse parvovirus (MPV), Rat minute virus (RMV), Rat parvovirus (RPV) or Rat virus (RV). A particularly preferred rat parvovirus is parvovirus H-1 (H-1PV).

In principle, any brain tumor (or possibly also brain metastases of other primary tumor origin) can be cured by intranasal application of a parvovirus, with gliomas, in particular glioblastomas, being preferred.

For intranasal administration, the parvovirus can be combined with suitable pharmaceutical carriers. Suitable pharmaceutical carriers of a type well known in the art and readily commercially available, include phosphate buffered saline (PBS) solutions, water, emulsions such as oil/water emulsions, wetting agents of various types, sterile solutions, etc. Such carriers can be formulated with the parvovirus by conventional formulating methods for administration to the subject at a suitable dose.

The dosage regimen of the parvovirus is readily determinable within the skill of the art, by the attending physician based on patient data, observations and other clinical factors, including for example the patient's size, body surface area, age, sex, the particular virus, vector, cell, etc. to be administered, the time and route of administration, the tumor type, grade, size etc., general health of the patient, and other drugs or therapies to which the patient is being subjected.

Preferably, intranasal administration is a single dose or three doses daily for three days.

Preferably, a single dose contains between 10⁷ and 10¹⁰ pfu.

Ranges between 10⁸ and 10⁹ pfu are particularly preferred.

The below examples explain the invention in more detail.

### Example 1

### Materials and Methods

### (A) Cells

The rat glioblastoma cell line RG-2 (kindly provided by C. Walz, University of Magdeburg, Germany) was grown in DMEM (Sigma-Aldrich Steinheim, Germany) supplemented with 10% FCS (Biochrom KG Berlin, Germany) and 1% antibiotics (penicillin, streptomycin; Gibco, Invitrogen Corporation Karlsruhe, Germany) in a 5% CO₂ humidified atmosphere at 37°C. Exponentially growing cells to be administered to rats were trypsinized and centrifuged (146 g/10 min), and the pellet was resuspended in DMEM without supplements.

### (B) H-1PV production and infection

H-1PV was amplified in human NB-K cells and purified on iodixanol *(5-{N-[3-(N-{3,5-bis[(2,3-dihydroxypropyl)carbamoyl]-2,4,6-triiodophenyl}acetamido)-2-hydroxypropyl]acetamido}-1-N,3-N-bis(2,3-dihydroxypropyl)-2,4,6-triiodobenzene-1,3-dicarboxamide,* GE Healthcare Canada Inc. Mississauga, ON) gradients as described previously (1). H-1PV was titrated on NB-K indicator cells by plaque assay and further used at multiplicities of infections expressed in plaque-forming units (pfu) per cell (2). Empty H-1PV particles were isolated by CsCl gradient centrifugation, and residual infectious particles were inactivated by UV-irradiation as described (3,4).

### (C) Animal Experiments

All animal experiments were carried out in accordance with institutional and state guidelines. Female Wistar inbred rats (10 weeks old; 26 in total), and immunodeficient RNU-rats (10 weeks old; 24 in total) were obtained from Charles River (Sulzfeld, Germany).

### (i) Intracerebral implantation of tumor cells

Rats were anesthetized with isoflurane (5% and 2,5% as initial and maintenance doses, respectively) and mounted to a stereotactic frame. After linear scalp incision, a 0.5-mm burr hole was made 2 mm right of the midline and 1 mm anterior to the coronal suture. The needle of a 10-µL Hamilton syringe was stereotactically introduced through the burr hole into the posterior part of the frontal lobe at a depth of 5 mm below the level of the *dura mater,* and RG-2 glioma cells or U87 human glioma cells, respectively (in each case 50,000 cells in a volume of 7 µL), were injected over 7 minutes. The needle was withdrawn slowly to minimize spreading of tumor cells along the needle tract.

### (ii) Intranasal H-1PV infection of glioma-bearing immunocompetent Wistar rats

At 7 days post RG-2 cell implantation, tumor-bearing rats were infected with H-1PV by intranasal application. Animals were shortly anesthetized as above and were instilled once with 10⁸ pfu of H-1PV in 100 µl PBS, 50 µL in each nostril. Control animals received either PBS or an equivalent number (1 x 10¹⁰) of non-infectious particles (UV-irradiated empty H-1PV capsids) in the same volume. Infected animals were kept separately from uninfected control rats under isolator conditions. Tumor growth was monitored every 4-5 days by MRI (see below). Clinical signs and weight of animals were monitored daily. Animals developing tumor-related symptoms or a body weight drop (>20%) were euthanized according to institutional guidelines.

### (iii) Magnetic resonance imaging (MRI)

The animals were examined in a 2.45-T MRI scanner (Bruker BioSpin MRI GmbH, Ettlingen, Germany) using T1-weighted imaging before and after injection of 0.4 mL contrast medium (Gadodiamide, Omniscan, Amersham, Freiburg) into the tail vein. Contrast-enhanced T1 imaging was performed 5 minutes after injection. During MR examination, rats were anesthetized by isoflurane insufflation (initial dose 5%, maintenance 2%, as above).

### (iv) Intranasal H-1PV infection of glioma-bearing immunodeficient RNU rats.

RNU rats with U87 xenografts were treated with H-1PV through multiple intranasal instillations, at days 8, 11, 14, 17, and 20 after tumor cell implantation. The total dose of H1-PV per animal was 5 x 10⁸ pfu.

### (D) Analyses of tissue samples

Animals from each group (glioma-bearing animals intranasally treated with PBS or with noninfectious [controls] or infectious H-1PV) were sacrificed 1, 3 and 5 days after mock or virus infection. Blood, brain and other organs (kidney, lung, heart, spleen, liver and gut) were analyzed for viral DNA.

### (i) DNA extraction from tissue samples

DNA was extracted from cryosections (10 µm thick) from shock-frozen specimens from different parts of both brain hemispheres (one bearing a tumor and the other not) and from samples of peripheral organs (see above; 25 mg of tissue) using the PureLink™ Genomic DNA Mini Kit (Invitrogen, Darmstadt, Germany). From complete blood, DNA was extracted with the QIA amp DSP blood kit (Quiagen, Hilden, Germany).
*(ii) PCR detection of H-1PV DNA.* PCR reactions were performed using 50 ng total DNA in 40 µl Supermix (Invitrogen, Darmstadt, Germany) and the following primers (0.2 µl of a 100 nM stock of each primer): sense primer, 5'-TCAATGCGCTCACCATCTCTG-3' (position nt 1996-2016 within the NS gene region of the H-1PV genome) and antisense primer, 5'-TCGTAGGCTTCGTCGTGTTCT-3' (position nt 2490-2510), yielding an amplified fragment of 516 bp. Amplification (34 cycles) was as follows: 3 min, 95°C starting reaction; cycle: 30 sec, 94°C; 30 sec, 58°C; 1 min, 72°C; final elongation 7 min, 72°C. Amplified fragments were separated by agarose (1.7%) gel electrophoresis at 120V. DNA from infected RG2 cells served as a positive control. Negative controls included H₂O and the corresponding tissue from a mock-infected rat.

### (iii) Dispersed cell assay

Filtered homogenates of brain and blood samples from H-1PV-infected animals were inoculated in triplicate on semiconfluent NB-K cells, seeded in 48-well plates. At the occurrence of the virus-mediated cytopathic effect (CPE), cells were harvested by trypsinization and pooled with the culture supernatant and trapped by suction onto GeneScreen (Perkin Elmer, Rodgau, Germany) transfer membrane or analysis of amplification of viral DNA using the dispersed cell assay as described previously (5). After cross-linking with UV (using the UV Stratalinker 1800 at 254 nm, Stratagene/Agilent, Waldbronn, Germany; auto crosslink program), filters were hybridized with a radio-labelled viral DNA probe kindly provided by J.F.P Nüesch and exposed to X-ray film (Amersham HyperFilm ECL, GE Healthcare, München, Deutschland).

### (E) Analysis of virus replication

Brains were removed from animals sacrificed 1 and 5 days after infection with H-1PV, and the two hemispheres were analyzed separately. After weighing, tissues were homogenized in 6 ml PBS in the presence of Matrix-D beads (Q-Biogene [Heidelberg, Germany]). One ml of this mixture was centrifuged, and supernatant was filtered trough 0.45 nm filters and used in serial dilutions (1:10 steps) for inoculation on NB-K and RG-2 indicator cells. When the controls reached confluence (4 d p.i.), cells were fixed with an acetate/ethanol mixture (1:3) and stained with crystal violet. Virus in the tissue was determined as the highest dilution that caused a CPE. (Titers were normalized to 1 g of brain tissue.)

### (F) Immunohistochemistry

For the immunodetection of parvoviral proteins, frozen 20 micrometer sections mounted on poly-L-lysine coated slides (Roth, Karlsruhe, Germany) were permeabilized with 0.1% Triton X-100 in PBS, and incubated with 5% goat serum in PBS for 1 h at room temperature to block non-specific staining. Sections were then incubated for 36 hours at 4°C with the mouse monoclonal NS-1 specific antibody 3d9 (courtesy of Dr. N. Salome) and/or the rabbit H-1PV-VP-specific antiserum (courtesy of Dr. C. Dinsart (6)). After rinsing, secondary antibodies (anti-mouse IgG conjugated with cyanin3 [red; Jackson ImmunoResearch, West Grove, USA], anti-mouse IgG conjugated with cyanin2 [green; GE Healthcare, Freiburg, Germany], or anti-rabbit IgG-conjugated with cyanin2 [Jackson ImmunoResearch, West Grove, USA]) were applied to the sections (3 h at room temperature) for revealing viral NS-1 and VP proteins, respectively. Finally, the coverslips were intensively washed and mounted with DAPI-containing medium (VESTASHIELD, Vector Laboratories, Burlingame, USA) to visualize the nuclei. Images were digitized using a Leica DMRBE fluorescent microscope (Leica, Bensheim, Germany) equipped with a digital camera and an image analysis software (analySIS, Olympus, Germany).

### (G) Histological analyses (H.-E. staining)

Frozen brain sections on slides were incubated for 3 min in haematoxylin (Sigma-Aldrich, Hamburg, Germany), washed with tape water, exposed for 5 min in eosin (Sigma-Aldrich) and incubated in increasing concentrations of ethanol (70%, 80%, 90% and 100%; 10 min for each concentration) followed by immersion in xylol (Merck, Darmstadt, Germany), covered using Eukitt (Kindler, Freiburg, Germany), and air-dried.

### (H) Virus-neutralizing antibodies

Blood samples were obtained from animals at different time points after H-1PV infection. Serial dilutions of the sera were made in MEM, and mixed with an equal volume of H-1PV suspension (corresponding to 8 x 10⁴ pfu/well). After incubation for 30 min at room temperature, the serum-virus mixture was inoculated on NB-K cells plated in 96-well plates (8 x 10³ cells/ well). After a 20 min incubation, cultures were supplemented with MEM (containing 5% heat-inactivated FCS and 1% penicillin-streptomycin), kept for 72 hours at 37°C and processed for the determination of the number of living cells (relative to mock-infected cultures) using the MTT assay. Briefly, MTT (3-[4,5 dimethylthiazol-2-yl]-2,5-diphenyltetrazolium bromide, provided by Sigma-Aldrich, USA) stock solution (5 mg/ml) was added to each culture (10 µL per 100 µL). After incubation for 3.5 h at 37°C, medium was removed, incorporated dye was solubilized with acidic isopropanol (100 µL/well), and extinction values were measured with a Multiskan EX apparatus (Thermo Electron Corporation, Dreieich, Germany) at 570 nm. The virus-neutralizing antibody titer was defined as the dilution of serum, which reduced the viral cytotoxic effect (i.e. fraction of living cells relative to mock-infected cultures) by 50%.

### (I) Statistics

Statistical significance of differences in survival rates between tumor-bearing animals that were infected with H-1PV versus mock-treated, was calculated using the logrank test, type Wilcoxon Rank sum test (7), modified for censored (sacrificed animals) survival data, and expressed as p-values. In the Kaplan-Meier curves, the sacrifice (i.e., censoring) time points are indicated by vertical bars.

### Example 2

### Treatment of glioma in a rat model using intranasal application of oncolytic parvovirus H-1 (H-1PV)

Intratumoral or intravenous infection with parvovirus H-1PV was recently shown to induce full regression of experimental gliomas in a rat model (8). Since there are hints that nasal epithelium might be a possible virus entry site in natural infection with parvoviruses (9), it was tested whether similar glioma suppression could be achieved by instilling H-1PV in the nasal cavity of animals. This was investigated in Wistar rats bearing intracranial RG-2 cell-derived (rat) gliomas and in (immunodeficient) RNU rats with human gliomas established from U87 cells. Fifty thousand cells were implanted stereotactically in the posterior part of the frontal lobe of the brains of 26 Wistar rats and 24 RNU rats, respectively, resulting in the formation of tumors. In the absence of further treatment, the animals had to be sacrificed because of tumor-related symptoms: survival of animals after tumor cell implantation was 22 days (RG-2 glioma) or 27 days (U87 glioma) at a maximum.

For treatment experiments, animals were infected with H-1PV by the intranasal route (10⁸ pfu in 100 µL, 50 µL in each nostril; one inoculation in 15 Wistar rats, multiple inoculations in 11 RNU rats) 7 days (Wistar rats) or 8, 11, 14, 17, and 20 days (RNU rats) after tumor cell implantation. Control groups were treated by instilling empty viral capsids (10¹⁰ particles in 100 µL; Wistar: n=5, RNU: n=7) or PBS (100 µL; Wistar, n=6; RNU, n=5) in the nostrils of the animals.

As apparent from MR images in Figure 1, a single intranasal instillation of H-1PV was sufficient to achieve complete remission of intracranial gliomas in 13 of 15 RG-2-derived glioma-bearing animals (Wistar rats). This striking effect required infectious virions since empty capsids failed to protect rats from glioma growth, with animals dying of their tumor after the same interval as PBS-treated controls, i.e. 12 to 22 days after glioma cell implantation (Fig. 2A). In the case of RNU rats bearing U87 cell-derived tumors, the tumor suppressive effect of H-1PV was less pronounced but infected animals survived one week longer than uninfected ones (none of the animals survived more than 32 days after tumor cell implantation, Fig 2B). Hence, in animals treated intranasally with infectious H-1PV, survival was significantly prolonged (RG-2 gliomas in Wistar rats, p=0.0021; U87 gliomas in RNU rats, p=0.0038) compared with animals that received PBS or inactive viral particles (Fig. 2 A,B). Survival probability was calculated by Kaplan-Meier analyses for the PBS-treated control group (Wistar rats, n=6; RNU rats, n=5) and for rats inoculated with empty viral particles (Wistar rats, n=5; RNU rats, n=7) or infectious H-1PV virions (Wistar rats, n=15; RNU rats, n=11). In the control groups, 2 animals were sacrificed for further analyses at day 3 p.i.. In the group treated with infectious H-1PV virions, animals were sacrificed for analyses at day 1 (2 Wistar rats, 1 RNU rat), 3 (4 Wistar rats, 1 RNU rat), 5 (2 Wistar rats, 2 RNU rats), and 45 (2 Wistar rats) following infection. Three surviving Wistar rats were sacrificed at day 78 p.i. to end experimentation (Fig. 2 A; 2 animals of the infected group died from tumor unrelated reasons, 18 days after tumor cell implantation). Sacrificed animals were included in the Kaplan-Meier calculation as censored animals. The highly efficient regression of RG-2-derived glioma could be confirmed in 3 additional independent experiments. Tumor regression after H-1PV infection was not accompanied by any adverse side effects in the animals (see also below).

Paralleling previous results with intratumoral or intravenous virus inoculation (8), intranasal infection with H-1PV induced destruction of tumor tissue (oncolysis) without leading to any morphological alteration in adjacent brain tissue (Fig. 3) or any other organ. However, it is to be noted that destruction was more efficient in RG-2 cell-derived tumors in immunocompetent animals compared to human xenografts (U87 cells) in immunodeficient animals (Fig. 3). This was also observed in previous studies using other than intranasal routes of infection (8).

Twenty-four hours after infection with full H-1PV virions, viral DNA could be detected by PCR or dispersed cell assay in the brain (Fig. 4 A,B) demonstrating that intranasally inoculated virus can reach brain tissue, with a predominant accumulation in the tumor area. A positive signal was also detected in the peripheral organs listed in Table 1 with relatively high signals in liver and spleen.

**Table 1**

| **Detection of viral DNA in blood and various organs of rats infected intranasally with H-1PV** | | | | | | | | |
|---|---|---|---|---|---|---|---|---|
| Rat strain | Days p.i.* | Blood | Kidney | Lung | Heart | Spleen | Liver | Gut |
| Wistar, with RG-2 glioma | 1 | + | + | - | - | + | + | + |
| | 3(5) | - | T | + | + | + | + | + |
| Wistar, without tumor | 1 | - | - | - | + | + | + | + |
| | 3(5) | - | + | - | - | + | + | + |
| RNU, with U87 glioma | 1 | + | + | + | + | + | + | + |
| | 3(5) | - | + | + | - | + | + | - |
| RNU, without tumor | 1 | - | + | - | + | + | + | + |
| | 3(5) | - | + | + | + | + | + | - |

| | | | | | | | | |
|---|---|---|---|---|---|---|---|---|
| DNA was extracted from organs and blood, and viral DNA was detected by PCR analysis or dispersed cell assay. * In the case of blood, the presence of viral DNA was analyzed at days 1 and 5, for organs at days 1 and 3 after infection. | | | | | | | | |

This shows that also after intranasal application, the virus got widely distributed as reported for intravenous and intracranial treatment (8) irrespective of the presence of tumor tissue in the animals. In blood, viral DNA could be detected in tumor bearing animals 1 day after infection bit not at days 3 and 5, indicating transient viremia. In cryosections of brain tissue, the viral cytotoxic nonstructural protein NS-1 that is required for viral DNA replication became detectable by immunohistochemistry starting from day 5 p.i., but exclusively in the tumor cell area (Fig. 5), and never in sections from peripheral organs (data not shown). Viral capsid (VP) proteins could be demonstrated at earlier times p.i. (3 days) in various brain sections, in particular in the tumor area (exemplified for RG-2 gliomas in Fig. 6) confirming the transfer to the brain of input virions as they were also detected in brain sections from animals having received empty viral capsids (data not shown). Infectious virions could be detected by inoculating brain tissue samples to indicator cells. Compared to the input of virus in the nostrils, virus titers in brain decreased with time due to rapid distribution to the whole body of the animal, irrespective of the presence of tumor tissue (data not shown).

Intranasal treatment of animals with infectious virions induced an antiviral immune response as evident from the appearance of H-1PV-neutralizing serum antibodies in immunocompetent (Wistar) rats, detectable around 10 days after infection (Fig. 7).

The data demonstrate that the intranasal route of administration of H-1PV is as successful in curing rat glioma in the RG-2 glioma model as the intracranial application (8), and superior to intravenous treatment which requires multiple injections of the virus (8). A favorable effect of intranasal H-1PV infection could also be demonstrated in immunodeficient animals with human U87 cell-derived gliomas.

### Conclusion

The present experiments show that intranasally administered H-1PV induced full regression of (RG-2 cell-derived) rat gliomas in about two weeks p.i.. Although this time interval was about one week longer compared with intracranial or intravenous infection (8), intranasally treated immunocompetent animals showed complete tumor suppression resulting in their long-term survival as evident from Kaplan-Meier analysis (significantly [p=0.0021] extended life span compared to mock-treated tumor-bearing rats). In immunodeficient RNU rats bearing a human (U87 cell-derived) glioma, a significantly prolonged survival was observed after infection with H-1PV compared to uninfected animals (p=0.0038).

The virus reached the brain and was distributed to other organs after intranasal instillation of H-1PV, as reported previously for intracranial and intravenous administration. This was evidenced by immunohistochemistry using an H-1PV capsid protein (VP)-specific antiserum, as well as by PCR analysis of viral DNA. An enrichment of input H-1PV capsids in the tumor area was observed, which has also been found for infectious virions in animals infected intravenously. Presence of viral DNA was transient with the notable exception of the tumor tissue. Expression of the regulatory and toxic viral protein NS-1 that is also necessary for virus replication could only be demonstrated within the tumor tissue and was not detected in other brain areas or organs from infected animals. Due to the early and efficient distribution of the virus throughout the whole body of the animals, titers of infectious virus dropped rapidly in specific areas compared to the amount of virus applied initially. Therefore, it is difficult to assess to which account the virus replicates yielding progeny virions remaining at the production site. However, since viral proteins necessary for replication are exclusively found in the tumor cells, it is most likely that replication takes place in these cells, spreading the virus within the tumor area and being distributed to other sites. Viremia seems to take place rather early after infection (within the first 24 hours), and may reappear in tumor-bearing animals at later stages, indicating virus replication in tumor cells, which is supported by the detection of the viral NS-1 protein in these cells. Altogether, these findings confirm the previously reported oncotropism of parvovirus replication. Detection of NS-1 protein in gliomas was delayed by 2 days compared to intravenous or intracranial application (8), in keeping with the delay in the onset of tumor regression observed by MR imaging.

Intranasal infection of immunocompetent rats led to induction of H-1PV-neutralizing antibodies after 10 days, which represents a delay of 3-4 days compared to the appearance of serum antibodies after intravenous or intracerebral administration (8). This might be an advantage in clinical applications of the virus when envisaging multiple infections.

In the present study it could be shown for the first time that H-1PV is able to reach the brain (and the tumor therein) as well as other organs when deposited on the nasal mucosa. Interestingly, virus from an infected glioma-bearing rat could be transmitted to an animal kept in another cage in the same isolator, correlating with the infection and full regression of the glioma carried by the latter. This observation suggests that virus (excreted via saliva or urine) can be transmitted by air to infect neighboring rats. It is tempting to speculate that nasal mucosa may be a site of virus entry.

The herein demonstrated efficacy of intranasal application of H-1PV in oncolytic virotherapy presents an advantage in clinical settings because it is an easy and safe-to-perform treatment not requiring invasive measures. As mentioned above, the relatively late appearance of serum antibodies against the virus may allow a wider time frame for multiple applications to reach a higher total virus dose.

### List of references

1. Wrzesinski C, Tesfay L, Salome N, Jauniaux JC, Rommelaere J, Cornelis J, et al. Chimeric and pseudotyped parvoviruses minimize the contamination of recombinant stocks with replication-competent viruses and identify a DNA sequence that restricts parvovirus H-1 in mouse cells. J Virol. 2003;77:3851-8.
2. Di Piazza M, Mader C, Geletneky K, Herrero YCM, Weber E, Schlehofer J, et al. Cytosolic activation of cathepsins mediates parvovirus H-1-induced killing of cisplatin and TRAIL-resistant glioma cells. J Virol. 2007;81:4186-98.
3. Paradiso PR. Analysis of the protein-protein interactions in the parvovirus H-1 capsid. J Virol. 1983;46:94-102.
4. Rayet B, Lopez-Guerrero JA, Rommelaere J, Dinsart C. Induction of programmed cell death by parvovirus H-1 in U937 cells: connection with the tumor necrosis factor alpha signalling pathway. J Virol. 1998;72:8893-903.
5. Schlehofer JR, Gissmann L, Matz B, zur Hausen H. Herpes simplex virus-induced amplification of SV40 sequences in transformed Chinese hamster embryo cells. Int J Cancer. 1983;32:99-103.
6. Kestler J, Neeb B, Struyf S, Van Damme J, Cotmore SF, D'Abramo A, et al. cis requirements for the efficient production of recombinant DNA vectors based on autonomous parvoviruses. Hum Gene Ther. 1999;10:1619-32.
7. Gart JJ, Krewski D, Lee PN, Tarone RE, Wahrendorf J. The Design and Analysis of Long-term Animal Experiments. In: IACR, editor. Statistical Methods in Cancer Research. Lyon: IARC; 1986.
8. Geletneky K, Kiprianova I, Ayache A, Koch R, Herrero y Calle M, Deleu L, et al. Regression of advanced rat and human gliomas by local or systemic treatment with oncolytic parvovirus H-1 in rat models. Neuro-Oncology. 2010;12:804-14.
9. Zee YC, MacLachlan NJ. Parvovridae and Circoviridae. In: Hirsh DC, MacLachlan NJ, Walker RL, editors. Veterinary Microbiology. 2nd ed: Blackwell Publishing; 2004. p. 301-11.

## Claims

1. A parvovirus for use in a method of treatment of a tumor and/or tumor metastases by intranasal administration, wherein the tumor is a brain tumor and/or the metastases are cerebral metastases.

2. A parvovirus for use in a method according to claim 1, wherein intranasal administration is a single dose once per day for 3 days.

3. A parvovirus for use in a method according to claim 2, wherein a single dose contains between 10⁷ and 10¹⁰ pfu.

4. A parvovirus for use in a method according to claim 3, wherein a single dose contains between 10⁸ and 10⁹ pfu.

5. A parvovirus for use in a method according to any one of claims 1 to 4 wherein the parvovirus is a rodent parvovirus.

6. A parvovirus for use in a method according to claim 5, wherein the rodent parvovirus is a rat parvovirus.

7. A parvovirus for use in a method according to claim 6, wherein the rat parvovirus is H1-PV.

8. A parvovirus for use in a method according to any one of claims 1-7, wherein the brain tumor is glioma.

9. A parvovirus for use in a method according to claim 8, wherein the glioma is a glioblastoma.

## Patentansprüche

1. Parvovirus zur Verwendung in einem Verfahren zur Behandlung eines Tumors und/oder von Tumormetastasen durch intranasale Verabreichung, wobei der Tumor ein Hirntumor ist und/oder die Metastasen zerebrale Metastasen sind.

2. Parvovirus zur Verwendung in einem Verfahren nach Anspruch 1, wobei die intranasale Verabreichung eine Einzeldosis einmal täglich für 3 Tage ist.

3. Parvovirus zur Verwendung in einem Verfahren nach Anspruch 2, wobei eine Einzeldosis zwischen 10⁷ und 10¹⁰ Pfu enthält.

4. Parvovirus zur Verwendung in einem Verfahren nach Anspruch 3, wobei eine Einzeldosis zwischen 10⁸ und 10⁹ Pfu enthält.

5. Parvovirus zur Verwendung in einem Verfahren nach einem der Ansprüche 1 bis 4, wobei das Parvovirus ein Nagetier-Parvovirus ist.

6. Parvovirus zur Verwendung in einem Verfahren nach Anspruch 5, wobei das Nagetier-Parvovirus ein Ratten-Parvovirus ist.

7. Parvovirus zur Verwendung in einem Verfahren nach Anspruch 6, wobei das Ratten-Parvovirus H1-PV ist.

8. Parvovirus zur Verwendung in einem Verfahren nach einem der Ansprüche 1-7, wobei der Hirntumor ein Gliom ist.

9. Parvovirus zur Verwendung in einem Verfahren nach Anspruch 8, wobei das Gliom ein Glioblastom ist.

## Revendications

1. Parvovirus destiné à être utilisé dans un procédé de traitement d'une tumeur et/ou de métastases tumorales par administration intranasale, dans lequel la tumeur est une tumeur du cerveau et/ou les métastases sont des métastases cérébrales.

2. Parvovirus destiné à être utilisé dans un procédé selon la revendication 1, dans lequel l'administration intranasale est une dose unique une fois par jour pendant 3 jours.

3. Parvovirus destiné à être utilisé dans un procédé selon la revendication 2, dans lequel une dose unique contient entre 10⁷ et 10¹⁰ pfu.

4. Parvovirus destiné à être utilisé dans un procédé selon la revendication 3, dans lequel une dose unique contient entre 10⁸ et 10⁹ pfu.

5. Parvovirus destiné à être utilisé dans un procédé selon l'une quelconque des revendications 1 à 4, dans lequel le parvovirus est un parvovirus de rongeur.

6. Parvovirus destiné à être utilisé dans un procédé selon la revendication 5, dans lequel le parvovirus de rongeur est un parvovirus de rat.

7. Parvovirus destiné à être utilisé dans un procédé selon la revendication 6, dans lequel le parvovirus de rat est HI-PV.

8. Parvovirus destiné à être utilisé dans un procédé selon l'une quelconque des revendications 1-7, dans lequel la tumeur cérébrale est un gliome.

9. Parvovirus destiné à être utilisé dans un procédé selon la revendication 8, dans lequel le gliome est un glioblastome.
